# EUROPÄISCHE PATENTANMELDUNG

(11) **EP 4 523 636 A1**
(43) Veröffentlichungstag der Anmeldung: **19.03.2025**
(21) Anmeldenummer: 23197140.9
(22) Anmeldetag: 13.09.2023
(51) Int. Cl.: A61B 17/221, A61B 17/00

(54) **VORRICHTUNG ZUR ENDOSKOPISCHEN ENTFERNUNG VON STEINEN ODER KONKREMENTEN AUS DEM GALLEN- UND PANKREASGANG UND VERFAHREN ZUR UMRÜSTUNG EINER IN EINEM ARBEITSKANAL EINES MEDIZINISCHEN ENDOSKOPS ANGEORDNETEN VORRICHTUNG**

(71) Anmelder: FUJIFILM Corporation, Tokyo 107-0052 (JP)
(72) Erfinder: Kelm, Danijel, 96138 Burgebrach (DE); Amon, Thassilo, 96129 Strullendorf (DE); Gorte, Igor, 91325 Adelsdorf (DE)
(74) Vertreter: Gosdin, Carstensen & Partner Patentanwälte Partnerschaftgesellschaft mbB

(57) **Zusammenfassung**

Eine Vorrichtung zur endoskopischen Entfernung von Steinen oder Konkrementen aus dem Gallen- und Pankreasgang weist einen in einem Tubus geführten Steuerdraht (3) auf, an dessen proximalem Ende ein Steuerelement zur axialen Verlagerung des Steuerdrahtes (3) innerhalb des Tubus und an dessen distalem Ende ein aus Korbdrähten bestehender Fangkorb befestigt sind. Dabei führt eine axiale Verlagerung des Steuerdrahts (3) mittels des Steuerelements zu einem schlingenartigen Aufweiten oder Zusammenziehen des Fangkorbes. Für eine derartige Vorrichtung sollen günstige Möglichkeiten zu deren Handhabung bei einer erforderlichen Lithotripsie geschaffen werden. Dabei ist der Steuerdraht (3) von dem Steuerelement trennbar und zur Lithotripsie des vom Fangkorb aufgenommenen Steins an ein Betätigungselement kuppelbar, mit dem eine ausreichende Kraft am Fangkorb erzeugbar ist.

## Beschreibung

Die Erfindung betrifft eine Vorrichtung zur endoskopischen Entfernung von Steinen oder Konkrementen aus dem Gallen- und Pankreasgang, die einen in einem Tubus geführten Steuerdraht aufweist, an dessen proximalem Ende ein Steuerelement zur axialen Verlagerung des Steuerdrahtes innerhalb des Tubus' und an dessen distalem Ende ein aus Korbdrähten bestehender Fangkorb befestigt sind, wobei eine axiale Verlagerung des Steuerdrahts mittels des Steuerelements zu einem schlingenartigen Aufweiten oder Zusammenziehen des Fangkorbes führt.

Weiterhin betrifft die Erfindung auch ein Verfahren zur Umrüstung einer in einem Arbeitskanal eines medizinischen Endoskops angeordneten Vorrichtung für eine Verwendung zur Lithotripsie und Entfernung eines von einem Fangkorb erfassten Steines, das nach einer endoskopischen retrograden Cholangiopankreatographie durchführbar ist, wobei die Vorrichtung einen Tubus, einen in diesem geführten Steuerdraht, einen distal mit dem Steuerdraht verbundenen Fangkorb zur Aufnahme eines Steines und ein am Tubus und am Steuerdraht angreifendes Steuerelement aufweist und wobei der Fangkorb durch eine axiale Verlagerung des Steuerdrahts mittels des Steuerelements schlingenartig aufgeweitet oder zusammengezogen wird.

Bei der endoskopischen retrograden Cholangiopankreatographie (ERCP) handelt es sich um ein endoskopisches Verfahren zur Darstellung und Untersuchung des Gallen- und Pankreasganges eines Patienten. Dabei wird ein medizinisches Endoskop über den Rachenraum des Patienten in dessen Speiseröhre eingeführt, wobei ein distales Ende des Endoskops mit einer als Seitenoptik ausgebildeten Kamera versehen ist und durch den Magen hindurch bis in den Zwölffingerdarm gelangt. Für eine weitere Untersuchung und eventuellen Behandlung der Gallen- und Pankreasgänge des Patienten werden unterschiedliche endoskopische Vorrichtungen, die distal mit Instrumenten versehen sind, über den Arbeitskanal des medizinischen Endoskops bis in den zu behandelnden Bereich der Gallen- und Pankreasgänge geführt. Mit diesen können verengte oder blockierte Gänge geöffnet, Gallensteine entfernt und/oder im Rahmen einer Lithotripsie zertrümmert, eine Biopsie und eine eventuelle operative Entfernung von Tumoren im Bereich der Gänge durchgeführt sowie Stents in die Gänge eingesetzt werden. Mittels der endoskopisch-retrograden Cholangiopankreatographie (ERCP) nach Papillotomie wird ein Gallenstein üblicherweise unter Verwendung eines Fangkorbes extrahiert. Der Fangkorb ist dabei auch für eine sichere Entfernung von Steinfragmenten aus dem Gallengang geeignet.

Eine Vorrichtung zur endoskopischen Entfernung von Steinen oder Konkrementen aus dem Gallen- und Pankreasgang der im Oberbegriff des Patentanspruchs 1 genannten Gattung ist aus der DE 10 2009 031 209 A1 bekannt. Diese weist ein in einem Tubus geführtes Seil auf, an dessen proximalen Ende ein Steuerelement zur axialen Verlagerung des Seils innerhalb des Tubus und an dessen distalen Ende ein aus zumindest vier elastischen Korbdrähten gebildeter Fangkorb angeordnet sind. Bei einer axialen Verlagerung des Seils mittels des Steuerelements wird der Fangkorb schlingenartig zusammengezogen oder aufgeweitet, da der Fangkorb gegen ein distales Ende des Tubus' und teilweise in dessen Lumen gezogen wird. Der Fangkorb dient zur Aufnahme eines aus der Papille gelösten Gallensteins und wird rotativ bewegt, um diesen zu erfassen, und verengt sich gleichzeitig. Der zwischen den Korbdrähten eingeklemmte Gallenstein ist in dem Fangkorb fixiert, um anschließend aus dem Gallen- und Pankreasgang entfernt zu werden.

Erhebliche Probleme treten stets dann auf, wenn die Abmessungen des Steins es nicht zulassen, diesen zu seiner Entfernung durch Körperöffnungen, wie z.B. den Gallenkanal zu ziehen. Eine gewaltsame Entfernung des Steins würde dann zu beträchtlichen Verletzungen des zu behandelnden Patienten führen. Um diesem Problem zu begegnen, bestehen nur die Möglichkeiten, den Fangkorb derart zu gestalten, dass über den Fangkorb eine Kraft auf den Stein zu dessen Lithotripsie ausgeübt wird oder dass der Fangkorb über die endoskopische Vorrichtung zur Freigabe des Steins betätigt werden kann, damit sich die Vorrichtung nach dem Trennen des Fangkorbs vom Stein aus dem Gallengang entfernen lässt.

Weiterhin ist aus der DE 32 16 178 A1 eine Vorrichtung zur mechanischen Lithotripsie und zur Entfernung von Steinen, die sich im Gallengang gebildet haben, bekannt. Diese Vorrichtung besteht aus einem Extraktor und kann bei Bedarf mit einer Einrichtung zur Lithotripsie kombiniert werden. Dabei weist der Extraktor eine Steuereinrichtung in Form eines mit einer Schubstange verbundenen Handgriffs und eine die Schubstange aufnehmende Hülse auf. Die Schubstange ist teleskopisch in der Hülse geführt und mit ihrem vom Handgriff abgewandten Ende an dem zum Steinfangkorb führenden Zugseil fixiert. Wird folglich die Schubstange axial in der Hülse verlagert, so führt das, je nach Bewegungsrichtung, zu einem Öffnen oder einem Schließen des Steinfangkorbes, so dass ein beispielsweise im Gallengang vorhandener Stein von dem Steinfangkorb aufgenommen und durch eine Bewegung der gesamten Vorrichtung in proximaler Richtung aus dem Körper des Patienten entfernt werden kann.

Zusätzlich zu dem Extraktor ist gemäß der DE 32 16 178 A1 ein separater Lithotriptor vorgesehen, der bei Bedarf an dem Stellelement fixiert wird. Es handelt sich dabei um ein Spannelement mit radial zu diesem verlaufenden Halterungen, die nach der Anbringung des Lithotriptors einerseits am Handgriff des ersten Stellelements und andererseits an einem mit der Hülse verbundenen Kopfstück angreifen. Wird festgestellt, dass der Stein Abmessungen besitzt, die dessen problemlose Entfernung verhindern, so wird über den an der Betätigungseinrichtung angesetzten Lithotriptor eine derart starke Zugkraft auf das Seil ausgeübt, dass der im Steinfangkorb befindliche Stein in mehrere kleine Partikel zerfällt. Diese Partikel lassen sich dann mit dem Steinfangkorb entfernen.

Es ist Aufgabe der vorliegenden Erfindung, für eine Vorrichtung zur endoskopischen Entfernung von Steinen oder Konkrementen aus dem Gallen- und Pankreasgang derart zu verbessern, dass sich günstigere Möglichkeiten von deren Handhabung in Bezug auf eine erforderliche Lithotripsie ergeben.

Diese Aufgabe wird, ausgehend vom Oberbegriff des Patentanspruchs mit dessen kennzeichnenden Merkmalen gelöst. Die vom Patentanspruch 1 abhängigen Patentansprüche beinhalten erfindungsgemäße Weiterbildungen dieser Lösung.

Danach ist eine Vorrichtung zur endoskopischen Entfernung von Steinen oder Konkrementen aus dem Gallen- und Pankreasgang vorgesehen, die einen in einem Tubus geführten Steuerdraht aufweist. An einem proximalen Ende des innerhalb des Tubus' verlaufenden Steuerdrahts ist ein Steuerelement zur axialen Verlagerung des Steuerdrahtes befestigt, während an dessen distalem Ende ein aus Korbdrähten bestehender Fangkorb befestigt ist. Dabei führt eine axiale Verlagerung des Steuerdrahts mittels des Steuerelements zu einem schlingenartigen Aufweiten oder Zusammenziehen des Fangkorbes. Über das Steuerelement können somit präzise Bewegungen ausgeführt werden, um den Stein zu erfassen und zu seiner Entfernung verliersicher zu umschließen.

Erfindungsgemäß soll der Steuerdraht von dem Steuerelement trennbar und zur Lithotripsie des vom Fangkorb aufgenommenen Steins an ein Betätigungselement kuppelbar sein, mit dem eine ausreichende Kraft am Fangkorb erzeugbar ist. Wenn, wie bereits dargelegt, der von dem Fangkorb aufgenommene Stein Abmessungen aufweist, die dessen Entfernung durch den Gallengang verhindern, so ist eine Lithotripsie des Steins erforderlich. Diese kann ausgeführt werden, indem auf den den Stein aufnehmenden Fangkorb, der über den Steuerdraht teilweise in das Lumen des Tubus' gezogen wird und sich somit verengt, eine derartige Kraft ausgeübt wird, dass der Stein, durch eine mechanische Lithotripsie zerdrückt, in eine Vielzahl von im Fangkorb verbleibende Steinfragmente zerfällt.

Eine dafür erforderliche Zugkraft kann mit dem am Steuerdraht angreifenden Steuerelement, das vorzugsweise für eine Einhandbedienung als Schieber mit einem Daumenring und zwei Fingerringen versehen ist, nicht aufgebracht werden. Bei der Lösung nach der Erfindung erfolgt ein Austausch des Steuerelements gegen ein für die anschließende Lithotripsie geeignetes Betätigungselement. Dafür wird das Steuerelement vom Steuerdraht getrennt und dieser ist anschließend mit dem Betätigungselement kuppelbar.

Demgegenüber ist nach der gattungsbildenden DE 10 2009 031 209 A1 mit der endoskopischen Vorrichtung zwar eine Entfernung eines Gallensteins mittels eines Fangkorbes vorgesehen; mit diesem wird aber keine mechanische Lithotripsie durchgeführt, da das Steuerelement nur zur Ausführung von Bewegungen sowie zum Aufweiten und Zusammenziehen des Fangkorbes vorgesehen ist. Daher können mit dem an der Vorrichtung vorgesehenen Steuerelement keine für eine mechanische Lithotripsie erforderliche Zugkraft an dem Fangkorb erzeugt werden.

Nach der weiteren zum Stand der Technik genannten DE 32 16 178 A1 ist zwar ein separater Lithotriptor vorgesehen, der aber bei Bedarf an dem Stellelement fixiert werden muss, damit sich mit diesem eine ausreichende Zugkraft am Steuerdraht erzielen lässt. Dieser Lithotriptor ist aber nicht für eine Kombination mit jeder Bauart von Steuerelementen geeignet. Insbesondere mit einem üblichen mit einer Hand zu bedienenden Steuerelement, das einen Daumenring und zwei Fingerringe aufweist, ist dieser Lithotriptor nicht kombinierbar.

Das Betätigungselement soll derart ausgebildet und dimensioniert sein, dass mit diesem für eine Lithotripsie des vom Fangkorb aufgenommenen Steins eine Zugkraft von bis zu 500N aufgebracht werden kann. Diese hohe Zugkraft, die am Fangkorb eine ausreichende Kraft zum Zerdrücken des Steins erzeugt, soll weder zu einer plastischen Verformung des Betätigungselements führen, noch dazu, dass dieses brüchig wird. Für eine Zertrümmerung eines Gallensteins von ca. 1cm Durchmesser wird eine Zugkraft von 200N benötigt.

In weiterer Ausgestaltung der Erfindung soll von dem Betätigungselement ein Zugmittel ausgehen, das über eine formschlüssige Verbindung mit dem Steuerdraht kuppelbar ist. Folglich wird der von dem Steuerelement getrennte Steuerdraht anschließend formsschlüssig mit dem Zugmittel verbunden. Es besteht dabei durch ein Ineinandergreifen von Verbindungspartnern dieser beiden Bauteile Zugmittel und Steuerdraht ein in Richtung der Zugkraft wirkender Formschluss.

In weiterer Ausgestaltung der Erfindung soll dass die formschlüssige Verbindung als Haken-Ösen-Verbindung ausgebildet sein. Der vorzugsweise am Steuerdraht ausgebildete Haken wird in diesem Fall in eine distale Öse des Zugmittels eingehängt. Eine Übertragung der mittels des Betätigungselements erzeugten hohen Zugkraft erfolgt somit unter Verwendung einfacher Mittel.

Dabei kann, wie weiterhin vorgesehen, der Steuerdraht nach seiner Trennung vom Steuerelement an seinem proximalen Endabschnitt mit einem Knick versehen werden, wobei der abgeknickte proximale Endabschnitt im Wesentlichen parallel zum Steuerdraht verläuft. Der Knick wirkt als Haken für die formschlüssige Verbindung mit der Öse, und es können Mittel vorgesehen sein, mit denen verhindert wird, dass der Haken unter der hohen Zugkraft die Öse freigibt.

Weiterhin sollen mit dem proximalen Endabschnitt Mittel zur Fixierung des Knicks zusammenwirken. Diese Mittel verhindern, dass sich der Knick unter der hohen, von der Öse auf diesen übertragenen Zugkraft aufbiegt. Vorzugsweise werden die Mittel dadurch geschaffen, dass der in die Öse des Zugmittels eingehängte proximale Endabschnitt des Steuerdrahts in ein Lumen eines aus Metall hergestellten Spiralschlauchs eingezogen wird, wodurch sich ein Aufbiegen des Knicks zuverlässig verhindern lässt. Ein Durchmesser des im Spiralschlauch ausgebildeten Lumens ist derart dimensioniert, dass sich zum einen der proximale Abschnitt in dieses einziehen und in diesem bewegen lässt und zum anderen eine radial wirkende Spannung auf den abgeknickten proximalen Endabschnitt, der die Öse aufnimmt, wirkt, so dass mit dieser Verbindung hohe Zugkräfte übertragbar sind.

Außerdem ist vorgesehen, dass das Betätigungselement einen in der gleichen Längserstreckung wie das Zugmittel verlaufenden Schaft mit einer quer zu diesem verlaufenden, an diesem drehbar gelagerten Spannvorrichtung aufweist, in die ein proximales Ende des Zugmittels einhängbar ist. Durch eine von der die Behandlung durchführenden Person an der Spannvorrichtung manuell erzeugte Drehbewegung wird das proximale Ende des als Draht oder Seil ausgebildeten Zugmittels auf die Spannvorrichtung, die spulenartig ausgebildet ist, aufgewickelt.

Bei einer weiteren Ausgestaltung der erfindungsgemäßen Vorrichtung soll der Knick im proximalen Endabschnitt des Steuerdrahts mittels eines Werkzeugs herstellbar sein, das zwei eine Winkelbewegung zueinander ausführende Werkzeugteile aufweist. Dabei kann das Werkzeug gemäß einer ersten Ausführungsform zwei über ein Scharnier miteinander verbundene Schenkel aufweisen, wobei der Steuerdraht an einem der Schenkel fixierbar ist. Vorzugsweise sind die Schenkel als flache, an einem ihrer Ränder über das Scharnier miteinander verbundene Kunststoffteile ausgebildet.

Gemäß einer zweiten Ausführungsform kann auch ein Werkzeug mit einem ringförmiges, Haltegriffe aufweisendes Basisteil mit einer in diesem drehbar geführten kreisförmige Scheibe vorgesehen sein. Der proximale Endabschnitt wird in diesem Fall über eine Einführöffnung des Basisteils radial sowohl in dieses als auch in einen sich daran anschließenden Mitnehmer der Scheibe eingeführt. Wird nun die Scheibe gegenüber dem Basisteil verdreht, so führt das zu einer hakenartigen Verformung des proximalen Endabschnitts.

Außerdem ist bei dem Werkzeug mit über das Scharnier verbundenen Schenkel vorgesehen sein, dass die Schenkel miteinander fluchtende Nuten zur Aufnahme des Steuerdrahtes aufweisen und dass zumindest an dem einen Schenkel eine Schelle zum Fixieren des Steuerdrahtes vorgesehen ist.

Weiterhin wird die der Erfindung zugrundeliegender Aufgabe auch im Rahmen eines Verfahrens zur Umrüstung einer in einem Arbeitskanal eines medizinischen Endoskops angeordneten Vorrichtung gelöst, die nach einer endoskopischen retrograden Cholangiopankreatographie verwendet wird, wobei die Vorrichtung zur Lithotripsie und Entfernung eines von einem Fangkorb erfassten Steines verwendet wird und einen Tubus, einen in diesem geführten Steuerdraht, einen distal mit dem Steuerdraht verbundenen Fangkorb zur Aufnahme eines Steines und einen am Tubus und am Steuerdraht angreifendes Steuerelement aufweist und wobei der Fangkorb durch eine axiale Verlagerung des Steuerdrahts mittels des Steuerelements schlingenartig aufgeweitet oder zusammengezogen wird,

Dabei sind erfindungsgemäß folgende Verfahrensschritte vorgesehen:
- Durchtrennen des Steuerdrahts in seinem proximalen Abschnitt, in dem dieser in einen Schaft des Steuerelements geführt ist;
- Knicken des Steuerdrahts in einem proximalen Endabschnitt zu einer hakenförmigen Ausbildung;
- Einhängen des proximal hakenförmigen Endabschnitts des Steuerdrahts in eine Öse eines von einem als Lithotriptor dienenden Betätigungselements;
- Bewegung der aus dem hakenförmigen Ende des Steuerdrahts und der Öse des Zugmittels durch Spannen des Betätigungselements in einen aus Metall hergestellten Spiralschlauch und
- weiteres Spannen des Betätigungselements zur Erzeugung einer Kraft am Fangkorb zum Zertrümmern des von diesem aufgenommenen Steines.

Weiterhin kann im Rahmen des Verfahrens zunächst nach dem Einhängen des mit dem Knick versehenen Endabschnitts zunächst händisch in proximaler Richtung gezogen werden, bis die Öse am distalen Ende des Spiralschlauchs anliegt. Dann wird das proximale Ende des Zugmittels mit einer am Betätigungselement angeordneten Spannvorrichtung verbunden und das Zugmittel anschließend über die Spannvorrichtung, die als Aufwickelspule ausgebildet ist, gespannt wird, bis der Spiralschlauch über die Öse geschoben ist. Anschließend wird durch eine weitere Betätigung der Spannvorrichtung die auf den Steuerdraht übertragene Zugkraft erhöht, bis der Fangkorb an das distale Ende des Tubus bewegt ist Schließlich wird durch eine weitere Erhöhung der Zugkraft der vom Fangkorb aufgenommene Stein zertrümmert.

Die Erfindung ist nicht auf die angegebene Kombination der Merkmale des unabhängigen Patentanspruchs 1 und der von diesem abhängigen Patentansprüche sowie der Merkmale des Verfahrensanspruchs 9 und des von diesem abhängigen Patentanspruchs 10 beschränkt. Es ergeben sich darüber hinaus weitere Möglichkeiten, einzelne Merkmale, insbesondere dann, wenn sie sich aus den Patentansprüchen, den zu diesen angegebenen Vorteilen, der nachfolgenden Beschreibung der Ausführungsbeispiele oder unmittelbar aus der Zeichnung ergeben, miteinander zu kombinieren. Außerdem soll die Bezugnahme der Patentansprüche auf die Zeichnung durch die Verwendung von Bezugszeichen den Schutzumfang der Patentansprüche auf keinen Fall auf die dargestellten Ausgestaltungsbeispiele beschränken.

Weitere Merkmale der Erfindung ergeben sich im Übrigen aus der nachfolgenden Beschreibung und aus der Zeichnung, in der ein Ausführungsbeispiel der Erfindung vereinfacht dargestellt ist.

Es zeigen:
- Figur 1: als Seitenansicht ein Steuerelement einer perspektivisch dargestellten Vorrichtung zur endoskopischen Entfernung von Steinen oder Konkrementen aus dem Gallen- und Pankreasgang, in der nur ein Teilabschnitt eines Tubus' sichtbar ist,
- Figur 2: als Seitenansicht einen distalen Endabschnitt des Steuerdrahts mit einem daran befestigten Fangkorb,
- Figur 3: eine Trennung des Steuerelements von dem Steuerdraht mittels eines Seitenschneiders, nachdem bei einem medizinischen endoskopischen Eingriff am Patienten festgestellt wurde, dass an dem vom Fangkorb aufgenommenen Stein mit erheblicher Kraft eine Lithotripsie durchgeführt werden muss,
- Figur 4: ein proximales Ende des Steuerdrahts, das in ein Werkzeug eingelegt ist, mit dem dessen Endabschnitt zu einem Haken gebogen wird,
- Figur 5: den zu einem Haken gebogenen proximalen Endabschnitt des Steuerdrahts,
- Figur 6: ein in einem metallischen Spiralschlauch angeordnetes Zugmittel, das an seinem distalen Ende eine Öse bildet und über diese mit dem Haken gekuppelt ist,
- Figur 7: ein zur Lithotripsie dienendes Betätigungselement, an dem ein proximales Ende des Zugmittels eingehängt wird,
- Figur 8: das gemäß der Figur 4 verwendete Werkzeug zum Knicken des proximalen Endabschnitts des Steuerdrahts in seinem Zustand, in dem der Steuerdraht in diesen eingelegt werden kann,
- Figur 9: das Werkzeug nach der Figur 8 in seinem geschlossenen Zustand, in dem die Knickung des Steuerdrahts bewirkt wird,
- Figur 10: eine alternative Ausführungsform eines Werkzeugs zum Knicken des Steuerdrahts und
- Figur 11: das gemäß der Figur 7 verwendete Betätigungselement.

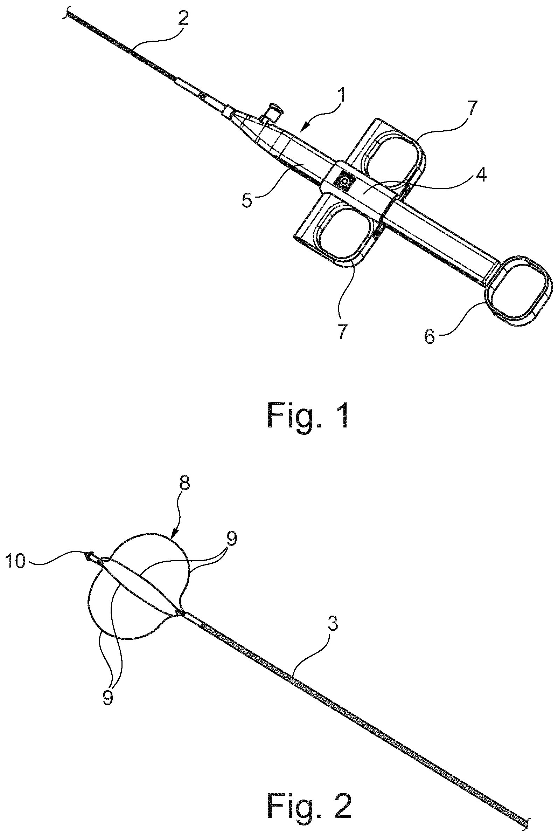

In der Figur 1 ist mit 1 ein Steuerelement bezeichnet, von dem ein Tubus 2 und ein in einem nicht näher dargestellten Lumen des Tubus` 2 verlaufender Steuerdraht 3, der der Figur 2 zu entnehmen ist, ausgehen. Das Steuerelement 1 weist einen mit dem proximalen Ende des Steuerdrahts 3 verbundenen Schieber 4 auf, der in einem nicht näher dargestellten Längsschlitz eines Führungsschafts 5 geführt ist. Dieser Führungsschaft 5 ist wiederum fest mit dem Tubus 2 verbunden, so dass sich mittels einer Relativbewegung des Schiebers 4 gegenüber dem Führungsschaft 5 der Steuerdraht 3 innerhalb des Tubus' 2 hin und her bewegen lässt. Zur Ausführung dieser Bewegungen ist der Führungsschaft 5 mit einem Daumenring 6 versehen, während der Schieber zwei Fingerringe 7 aufweist.

Die Figur 2 zeigt einen distalen Endabschnitt des Steuerdrahts 3 ohne den diesen umgebenden Tubus 2, wobei am distalen Ende ein Fangkorb 8 fixiert ist. Korbdrähte 9 dieses in der Darstellung geöffneten Fangkorbs 8 sind an ihren jeweiligen Enden gebündelt, und zwar einerseits am Ende des Steuerdrahts 3 und andererseits in einem das distale Ende des Fangkorbs 8 bildenden patronenförmigen Führungselement 10.

Der Tubus 2 mitsamt dem Steuerdraht 3 und dem Fangkorb 8 wird vorzugsweise in den Arbeitskanal eines über den Rachenraum, die Speiseröhre und den Magen eines Patienten bis in den Bereich des Gallen- und Pankreasganges eines Patienten verlaufenden medizinischen Endoskops eingeführt, das nicht dargestellt ist. Dabei befindet sich der Fangkorb 8 zunächst in seiner geschlossenen Stellung, in welcher er völlig von dem Tubus 2 aufgenommen ist. Über einen vorzugsweise in ein zweites Lumen des Tubus' 2 einführbaren Führungsdraht, der in die Körperöffnung eingeführt und unter endoskopischer oder radiologischer Betrachtung bis in den Bereich eines vorhanden Steines verläuft, wird der den Steuerdraht 3 und den Fangkorb 8 aufnehmende Tubus 2 nachgeführt.

Anschließend wird der Fangkorb 8 über das Steuerelement 1 in seinen in der Figur 2 geöffneten Zustand betätigt, wobei das aufgrund der Eigenspannung des Fangkorbs 8 geschieht. In dieser geöffneten Stellung kann der mittels des Steuerelements 1 positionierte sowie geöffnete Fangkorb 8 den Stein aufnehmen. Vorzugsweise führt der Fangkorb 8 während der Aufnahme des Steins und seiner anschließenden über das Steuerelement 1 bewirkten Schließbewegung, bei der sich der Fangkorb 8 am distalen Ende des Tubus' 2 abstützt, eine Drehbewegung aus, wodurch das Aufnehmen des Steins vereinfacht wird. Bei einer anschließenden Zugbewegung des relativ zum Tubus 2 bewegten Steuerdrahts 3 zieht sich der Fangkorb 8 fest um den Stein zusammen und umschließt diesen, so dass dieser, über den Steuerdraht 3 und den Tubus 2 in dem Fangkorb 8 verspannt, die entsprechenden Körperöffnungen passierend, sicher entfernt werden könnte.

Erhebliche Probleme treten aber dann auf, wenn der Stein derartige Abmessungen besitzt, dass er nicht durch Körperöffnungen, wie z.B. den Gallenkanal gezogen werden kann. Eine gewaltsame Entfernung des Steins würde zu beträchtlichen Verletzungen des zu behandelnden Patienten führen. Mit dem Steuerelement 1, das primär zur Lokalisierung und zum Aufnehmen des Steins geeignet ist, kann aber auch keine ausreichende Zugkraft über den Steuerdraht 3 auf den Fangkorb 8 übertragen werden, um den Stein zu zertrümmern. Daher bleibt nur die Möglichkeit, den Fangkorb 8 über eine an diesem vorgesehene Auslöseeinrichtung zu öffnen, um den Stein freizugeben. Dann müssen aber die vorangegangenen Vorgänge wiederholt werden, um den Stein mit einem Korb eines separaten Lithotriptors aufzunehmen.

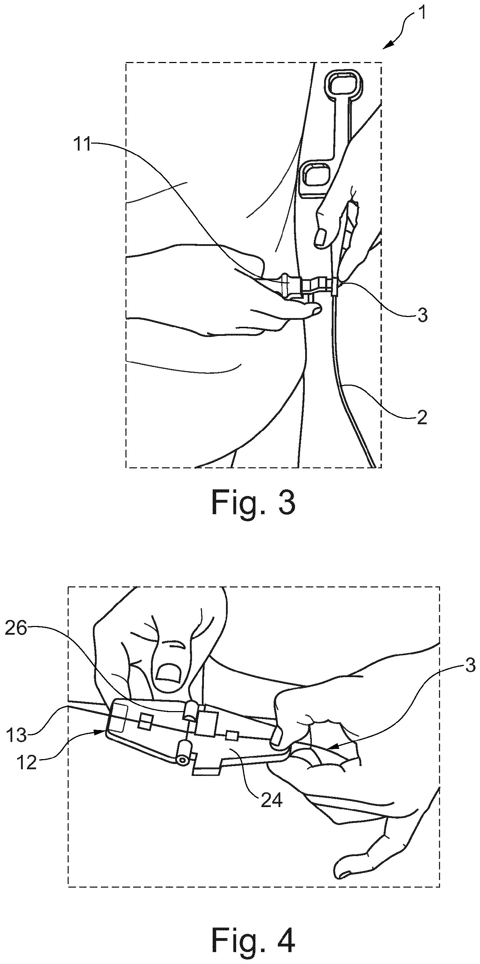

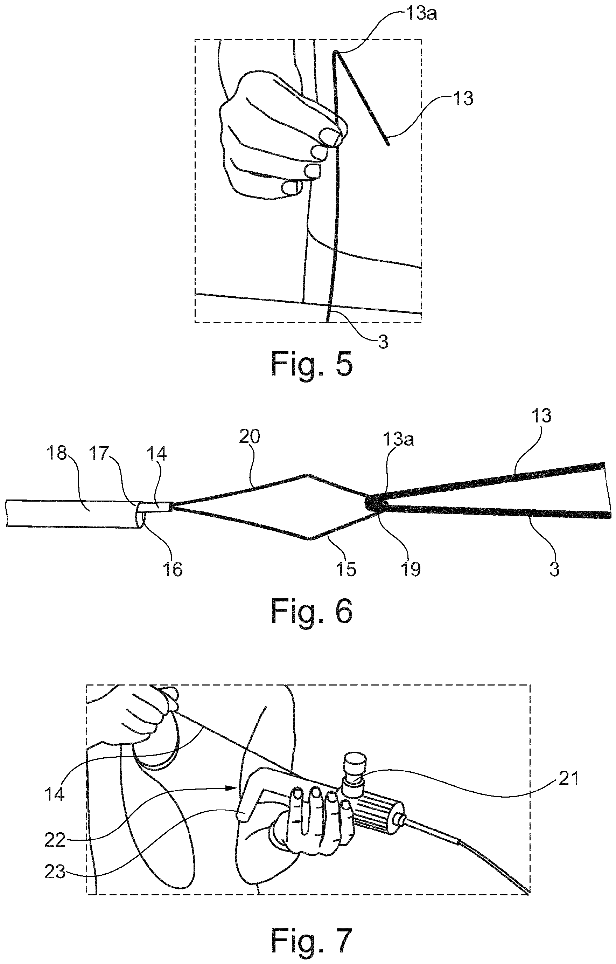

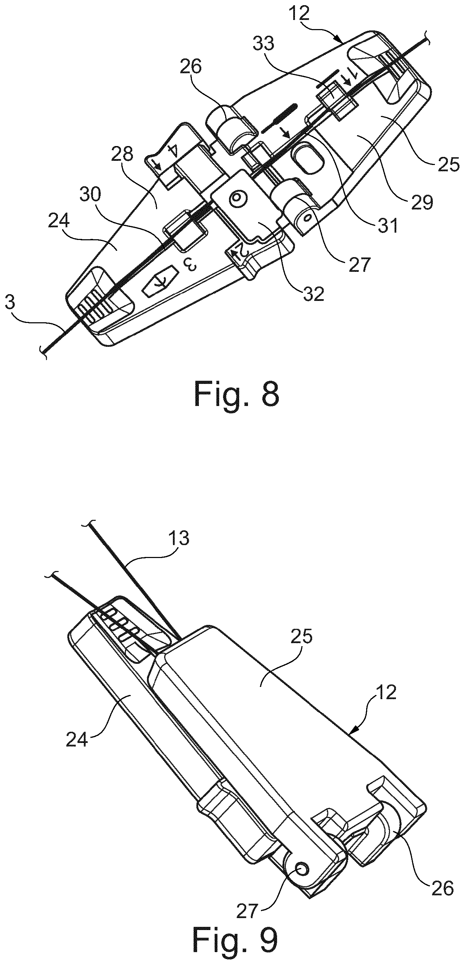

Erfindungsgemäß wird dann, wenn eine Lithotripsie des für eine normale Entfernung zu großen Steins erforderlich ist, wie der Figur 3 zu entnehmen ist, der Tubus 2 und der Steuerdraht 3 proximal mittels eines Seitenschneiders 11 vom Steuerelement 1 getrennt. Die Trennung des Steuerdrahts 3 erfolgt derart, dass von diesem ein proximaler Endabschnitt 13 noch axial über das proximale Ende des Tubus 2 vorsteht. Wie aus der Figur 4 hervorgeht, wird dieser vorstehende Endabschnitt 13 des Steuerdrahts 3 in ein Werkzeug 12 eingelegt und, wie nachfolgend im Zusammenhang mit der Figur 8 noch näher erläutert werden wird, in diesem Werkzeug 12 fixiert, Mit dem Werkzeug 12 wird ein proximaler Endabschnitt 13, der in der Figur 5 dargestellt ist, hakenförmig umgebogen, wodurch im proximalen Endabschnitt 13 ein Knick 13a entsteht..

Der hakenförmig umgebogene Endabschnitt 13 wird, wie in der Figur 6 dargestellt, in eine distal an einem als Draht oder Seil ausgebildeten Zugmittel 14 vorgesehene Öse 15 eingehängt, die eine rautenartige Außenkontur aufweist. Das Zugmittel 14 verläuft in einem Lumen 16 eines aus Metall hergestellten Spiralschlauchs 17, der in einem Tubus 18 angeordnet ist. Durch die rautenartige Außenkontur der Öse 15 wird erreicht, dass sich der in diese eingehängte hakenförmige Endabschnitt des Steuerdrahts 3 in einem distal spitzwinklig verlaufenden Bereich 19 der Öse 15 fängt und Ösendrähte 20 radial zusammengezogen werden, wenn die Öse 15 in das Lumen 16 gezogen wird.

Gemäß der Figur 7 wird das Zugmittel 14 händisch in proximaler Richtung gezogen und dann in eine Spannvorrichtung 21 eines Betätigungselements 22 eingehängt. Dabei erstreckt sich die Spannvorrichtung 21 rechtwinklig zu einem Griffteil 23 des Betätigungselements 22 und ist an diesem drehbar gelagert. Wird somit die Spannvorrichtung 21 in der Weise manuell betätigt, dass sie eine Drehbewegung gegenüber dem Griffteil 23 ausführt, so wird damit bewirkt, dass das Zugmittel 14 an einem entsprechend ausgebildeten Abschnitt der Spannvorrichtung 21 aufgewickelt wird.

Die Folge davon ist, dass die am distalen Ende des Tubus 18 gemäß der Figur 6 über diesen vorstehende Öse 15 mitsamt des in diese eingehakten proximalen Endabschnitts 13 des Steuerdrahts 3 in das Lumen 16 der Spiralschlauch 17 gezogen wird. Dadurch tritt eine zunehmende Zugkraft sowohl im Zugmittel 14 als auch in dem mit diesem gekuppelten Steuerdraht 3 auf, die auf den Fangkorbs 8 zur Lithotripsie des entsprechenden Steins übertragen wird.

Die weiteren Figuren 8 und 9 zeigen in unterschiedlichen perspektivischen Darstellungen das zum Biegen des Steuerdrahts 3 verwendete Werkzeug 12, dessen Funktion bereits im Zusammenhang mit der Figur 4 erläutert wurde. Das Werkzeug 12 besteht aus zwei schwenkbar zueinander geführten Schenkeln 24 und 25, die über Scharniere 26 und 27 miteinander verbunden sind. In der Figur 8 befindet sich das Werkzeug 12 in einem geöffneten Zustand, wobei Flächen 28 und 29 der beiden Schenkel 24 und 25 gezeigt werden, die beim Biegen eines Steuerdrahts 3, geführt an den Scharnieren 26 und 27, aufeinander zu bewegt werden.

In den Flächen 28 und 29 der Schenkel 24 und 25 befinden sich miteinander fluchtende Nuten 30 und 31, in die der Steuerdraht 3 vor dem Biegevorgang eingelegt wird. Anschließend wird der Steuerdraht 3 über ein an dem Hebelelement 24 vorgesehenes Federblech 32 und eine an dem Hebelelement 25 angeordnete Klammer 33 in seiner in den beiden Nuten 30 und 31 befindlichen Position gehalten. Anschließend wird das Werkzeug 12, wie in der Figur 9 dargestellt, zusammengeklappt, so dass der von diesem aufgenommene proximale Endabschnitt 13 des Steuerdrahts 3 zu dem in der Figur 5 dargestellten hakenförmigen Verlauf verformt ist.

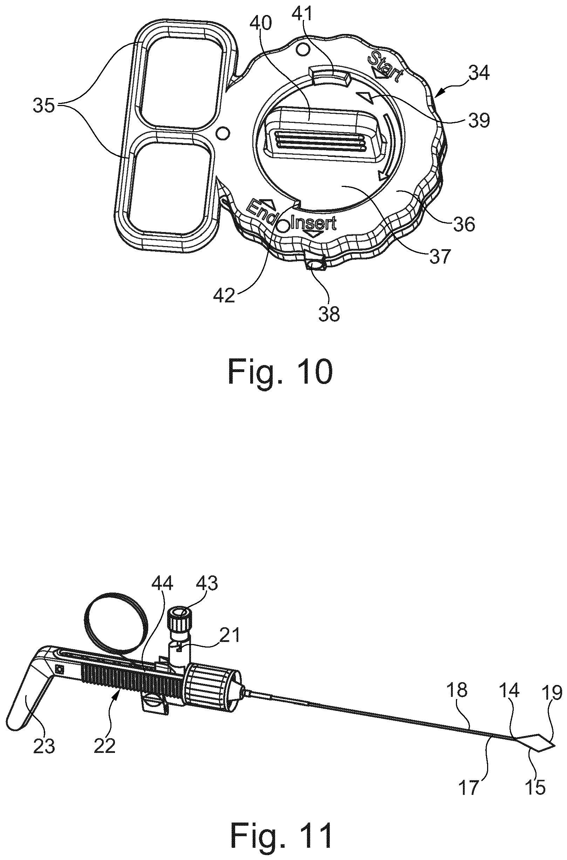

In der Figur 10 ist eine alternative Ausführungsform eines Werkzeugs 34 dargestellt, das aus einem ringförmigen, mit Haltegriffen 35 versehenen Basisteil 36 und einer in diesem drehbar geführten kreisförmigen Scheibe 37 besteht. Der in der Figur 4 dargestellte proximale Endabschnitt 13 wird in diesem Fall über eine Einführöffnung 38 des Basisteils 36 sowohl in dieses als auch in einen sich daran anschließenden, nicht näher dargestellten Kanal der Scheibe 37 eingeführt.

Das geschieht in einer Stellung der Scheibe 37 zum Basisteil 36, in der ein Pfeil 39 auf eine Markierung "Start" zeigt. Über einen Betätigungshebel 40 an der Scheibe 37 wird diese anschließend aus dieser Position heraus im Uhrzeigersinn in eine durch Endanschläge 41 und 42 begrenzte Endposition verdreht. Dadurch wird der zwischen dem Basisteil 36 und der Scheibe 37 geführte Teil des eingelegten Steuerdrahts 3 geknickt.

Schließlich zeigt die Figur 11 das bereits im Zusammenhang mit der Figur 7 erläuterte Betätigungselement 22, in dessen Spannvorrichtung 21 das Zugmittel 14 eingehängt ist. Wenn das an der Spannvorrichtung 21 ausgebildete, rechtwinklig zu einem Schaft 44 verlaufende Griffteil 43, das mit einer Rändelung versehen ist, verdreht wird, führt das zu einer Verlagerung des Zugmittels 14 innerhalb des Tubus 18 in proximaler Richtung, wodurch die über das distale Ende des Tubus vorstehende Öse 15 in den im Tubus 18 angeordneten Spiralschlauch 17 eingezogen wird.

### Bezugszeichenliste

- 1: Steuerelement
- 2: Tubus
- 3: Steuerdraht
- 4: Schieber von 1
- 5 5: Führungsschaft von 1
- 6: Daumenring von 1
- 7: Fingerring von 1
- 8: Fangkorb
- 9: Korbdrähte von 8
- 10: patronenförmiges Führungselement von 8
- 11: Seitenschneider
- 12: Werkzeug
- 13: proximaler Endabschnitt von 3
- 13a: Knick in 13
- 14: Zugmittel
- 15: Öse
- 16: Lumen
- 17: Spiralschlauch
- 18: Tubus
- 19: distal spitzwinklig verlaufenden Bereich von 15
- 20: Ösendraht
- 21: Spannvorrichtung
- 22: Betätigungselement
- 23: Griffteil
- 24: Hebelelement
- 25: Hebelelement
- 26: Scharnier
- 27: Scharnier
- 28: Fläche von 24
- 29: Fläche von 5
- 30: Nut in 28
- 31: Nut in 29
- 32: Federblech
- 33: Klammer
- 34: Werkzeug
- 35: Haltegriffe
- 36: Basisteil
- 37: kreisförmige Scheibe
- 38: Einführöffnung
- 39: Pfeil
- 40: Betätigungshebel
- 41: Endanschlag an 37
- 42: Endanschlag an 36
- 43: Griffteil von 21
- 44: Schaft von 22

## Patentansprüche

1. Vorrichtung zur endoskopischen Entfernung von Steinen oder Konkrementen aus dem Gallen- und Pankreasgang, die einen in einem Tubus (2) geführten Steuerdraht (3) aufweist, an dessen proximalem Ende ein Steuerelement (1) zur axialen Verlagerung des Steuerdrahtes (3) innerhalb des Tubus (2) und an dessen distalem Ende ein aus Korbdrähten (9) bestehender Fangkorb (8) befestigt sind, wobei eine axiale Verlagerung des Steuerdrahts (3) mittels des Steuerelements (1) zu einem schlingenartigen Aufweiten oder Zusammenziehen des Fangkorbes (8) führt, **dadurch gekennzeichnet, dass** der Steuerdraht (3) von dem Steuerelement (1) trennbar und zur Lithotripsie des vom Fangkorb (8) aufgenommenen Steins an ein Betätigungselement (22) kuppelbar ist, mit dem eine ausreichende Kraft am Fangkorb (8) erzeugbar ist.

2. Vorrichtung nach Patentanspruch 1, **dadurch gekennzeichnet, dass** mit dem Betätigungselement (22) eine Zugkraft von bis zu 500N aufbringbar ist, um den Stein zu zertrümmern.

3. Vorrichtung nach Patentanspruch 1, **dadurch gekennzeichnet, dass** von dem Betätigungselement (22) ein Zugmittel (14) ausgeht, das über eine formschlüssige Verbindung mit dem Steuerdraht (3) kuppelbar ist.

4. Vorrichtung nach Patentanspruch 3, **dadurch gekennzeichnet, dass** die formschlüssige Verbindung als Haken-Ösen-Verbindung (13 und 15) ausgebildet ist.

5. Vorrichtung nach Patentanspruch 4, **dadurch gekennzeichnet, dass** der Steuerdraht (3) nach seiner Trennung vom Steuerelement (1) an seinem proximalen Endabschnitt (13) mit einem Knick (13a) versehen wird, wobei der abgeknickte proximale Endabschnitt (13) unter einem spitzen Winkel oder parallel zum Steuerdraht (3) verläuft.

6. Vorrichtung nach Patentanspruch 5, **dadurch gekennzeichnet, dass** mit dem proximalen Endabschnitt (13) Mittel zur Fixierung des Knicks (13a) zusammenwirken.

7. Vorrichtung nach Patentanspruch 5, **dadurch gekennzeichnet, dass** der in die Öse (15) des Zugmittels (14) eingehängte proximale Endabschnitt (13) des Steuerdrahts (3) gemeinsam mit der Öse (15) in ein Lumen (16) eines aus Metall hergestellten Spiralschlauchs (17) eingezogen wird und als Mittel zur Fixierung des Knicks (13a) dient.

8. Vorrichtung nach Patentanspruch 3, **dadurch gekennzeichnet, dass** das Betätigungselement (22) einen in der gleichen Längserstreckung wie das Zugmittel (14) verlaufenden Schaft (44) mit einer quer zu diesem verlaufenden, an diesem drehbar gelagerten Spannvorrichtung (21) aufweist, in die ein proximales Ende des Zugmittels (14) einhängbar ist.

9. Vorrichtung nach Patentanspruch 5, **dadurch gekennzeichnet, dass** der Knick (13a) im proximalen Endabschnitt (13) des Steuerdrahts (3) mittels eines Werkzeugs (12, 34) herstellbar ist, das zwei eine Winkelbewegung zueinander ausführende Werkzeugteile (24 und 25, 36 und 37) aufweist.

10. Vorrichtung nach Patentanspruch 9, **dadurch gekennzeichnet, dass** die Werkzeugteile als zwei über ein Scharnier miteinander verbundene Schenkel (24 und 25) ausgebildet sind, wobei der Steuerdraht (3) an einem der Schenkel (24) fixierbar ist.

11. Vorrichtung nach Patentanspruch 10, **dadurch gekennzeichnet, dass** die Schenkel (24 und 25) miteinander fluchtende Nuten (30 und 31) zur Aufnahme des Steuerdrahtes (3) aufweisen und dass an zumindest einem der Schenkel (30, 31) eine Schelle (32, 33) zum Fixieren des Steuerdrahtes (3) vorgesehen ist.

12. Verfahren zur Umrüstung einer in einem Arbeitskanal eines medizinischen Endoskops angeordneten Vorrichtung für eine Verwendung zur Lithotripsie und Entfernung eines von einem Fangkorb (8) erfassten Steines, das nach einer endoskopischen retrograden Cholangiopankreatographie durchführbar ist, wobei die Vorrichtung einen Tubus (2), einen in diesem geführten Steuerdraht (3), einen distal mit dem Steuerdraht (3) verbundenen Fangkorb (8) zur Aufnahme eines Steines und ein am Tubus (2) und am Steuerdraht (3) angreifendes Steuerelement (3) aufweist und wobei der Fangkorb (8) durch eine axiale Verlagerung des Steuerdrahts (3) mittels des Steuerelements (1) schlingenartig aufgeweitet oder zusammengezogen wird, **gekennzeichnet durch** folgende Verfahrensschritte:
- Durchtrennen des Steuerdrahts (3) in seinem proximalen Abschnitt, in dem dieser in einen Führungsschaft (5) des Steuerelements (1) geführt ist;
- Knicken des Steuerdrahts (3) in einem proximalen Endabschnitt (13) zu einer hakenförmigen Ausbildung;
- Einhängen des proximal hakenförmigen Endabschnitts (13) des Steuerdrahts (3) in eine Öse (15) eines von einem zur Lithotripsie dienenden Betätigungselements (22);
- Bewegung der aus dem hakenförmigen Ende (13) des Steuerdrahts (3) und der Öse (15) des Zugmittels (14) in Längsrichtung in einen aus Metall hergestellten Spiralschlauch (17) durch Spannen des Betätigungselements (22) und
- weiteres Spannen des Betätigungselements (22) zur Erzeugung einer Kraft am Fangkorb (8) zum Zertrümmern des von diesem aufgenommenen Steines.

13. Verfahren nach Patentanspruch 12, **dadurch gekennzeichnet, dass** nach dem Einhängen des mit dem Knick (13a) versehenen Endabschnitts (13) zunächst händisch in proximaler Richtung gezogen wird, bis die Öse am distalen Ende des Spiralschlauchs (17) anliegt, dass das proximale Ende des Zugmittels (14) mit einer am Betätigungselement (22) angeordneten Spannvorrichtung (21) verbunden und das Zugmittel (14) anschließend über die Spannvorrichtung (21), die als Aufwickelspule ausgebildet ist, gespannt wird, bis der Spiralschlauch (17) über die Öse (15) geschoben ist, dass anschließend durch eine weitere Betätigung der Spannvorrichtung die auf den Steuerdraht (3) übertragene Zugkraft erhöht wird, bis der Fangkorb (8) an das distale Ende des Tubus (2) bewegt ist, und dass durch eine weitere Erhöhung der Zugkraft der vom Fangkorb (8) aufgenommene Stein zertrümmert wird.
